# EUROPEAN PATENT SPECIFICATION

(11) **EP 0 707 483 B1**
(45) Date of publication and mention of the grant of the patent: **09.04.2003**
(21) Application number: 94920809.4
(22) Date of filing: 23.06.1994
(51) Int. Cl.: A61K 31/765, A61K 31/785, A61L 15/58

(54) **USE OF CYANOACRYLATE ADHESIVES FOR THE MANUFACTURE OF FORMULATIONS TO INHIBIT SKIN ULCERATION**
VERWENDUNG VON CYANOACRYLATKLEBSTOFFEN ZUR HERSTELLUNG VON MITTELN ZUR VERHINDERUNG VON HAUTULZERATIONEN
L'UTILISATION DE CYANO-ACRYLATES SOUS FORME D'ADHESIFS POUR LA PREPARATION DE COMPOSITIONS EMPECHANT L'ULCERATION DE LA PEAU

(30) Priority: 25.06.1993 US 82927
(43) Date of publication of application: 24.04.1996
(73) Proprietor: Flowers Park Limited, Cheshire, CW7 3PD (GB)
(72) Inventor: TIGHE, Patrick J., Littleton, CO 80120 (US); BYRAM, Michael M., Colorado Springs, CO 80906 (US); BARLEY, Leonard V., Jr., Colorado Springs, CO 80906 (US); GREFF, Richard J., Yorba Linda, CA 92687 (US)
(74) Representative: Atkinson, Peter Birch
(86) International application number: PCT/US94/07153
(87) International publication number: WO 95/000153

(56) References cited:
- WO-A-93/15746
- WO-A-93/20828
- US-A- 2 804 073
- US-A- 3 667 472
- US-A- 3 995 641
- US-A- 4 035 334
- US-A- 4 287 177
- US-A- 5 270 168
- PATENT ABSTRACTS OF JAPAN vol. 007, no. 277 (C-199), 9 December 1983 (1983-12-09) & JP 58 157717 A (NEO SEIYAKU KOGYO KK), 19 September 1983 (1983-09-19)

## Description

### BACKGROUND OF THE INVENTION

### Field of the Invention

This invention is directed to the use of cyanoacrylate adhesives for the manufacture of a cyanoacrylate adhesive formulation for inhibiting the formation of skin ulcers. The cyanoacrylate adhesive to be used can be stored in dispensers for single or repeated/intermittent use and can be applied to the skin by spraying, painting, etc. of the adhesive.

### State of the Art

Cyanoacrylate adhesives have been suggested for a variety of adhesive purposes including glues and adhesives for surgical treatments. In particular, cyanoacrylate adhesives of formula I: 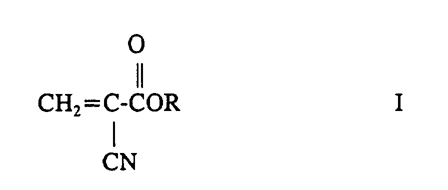 wherein R is an alkyl or other suitable substituents are disclosed in U.S. Patent Nos. 3,527,224; 3,591,676; 3,667,472; 3,995,641; 4,035,334; and 4,650,826. Typically, when used as adhesives for treating living tissues, the R substituent is alkyl of from 2 to 8 carbon atoms and most often is butyl (e.g., n-butyl).

The suggested medical uses for cyanoacrylate adhesives include surgical environments wherein the cyanoacrylate adhesives are utilized to adhere internal tissues, repair surgical wounds and mitigate bleedings e.g., as an alternative to sutures or as a hemostat. Typically, treatment is limited to a small defined broken skin area.

In contrast to such prior art uses of cyanoacrylate adhesives, this invention is directed to uses of cyanoacrylate adhesives for the manufacture of cyanoacrylate adhesive formulations for inhibiting the formation of skin ulcers including, by way of example, decubitus ulcers (bedsores) and diabetic ulcers by topical application of cyanoacrylate adhesives on intact broken skin.

Decubitus ulcers arise from the deprivation of nutrients to the tissue arising from prolonged pressure which leads to ischemia and are common in situations were the patient remains in a fixed position for prolonged periods (e.g., long term bed or wheelchair confinement). This often occurs in tissue overlying bony prominences. However, other factors such as skin irritation (due to, for example, friction and shearing forces arising from bedding materials and clothing), moisture (bodily fluid contamination), and poor skin integrity, especially in older, compromised patients, can exacerbate and accelerate ulcer formation and skin breakdown. The effect of friction in addition reduces the amount of pressure needed for skin breakdown. When the skin becomes broken, the potential for infection and morbidity increase significantly. Care and handling of the ulcerated area become more difficult and expensive. Typically, decubitus ulcer formation is preceded by reddening of nutrient deprived skin which, with continued irritation, develops into the open bedsore (i.e., a skin ulcer).

Diabetic ulcers are formed by deprivation of nutrients to the tissue as a result of the diabetic condition including neuropathy (lack of pain sensation), poor circulation in the patient, etc. In particular, diabetic ulcer formation in nutrient deprived tissues is facilitated by skin irritation much as with decubitus ulcer formation due to, for example, bodily fluid contamination, friction, shearing forces, and poor skin integrity. Typically, diabetic ulcer formation is preceded by reddening of nutrient deprived tissue which, with continued irritation, develops into an open skin ulcer.

In any event, once formed, skin ulceration is prone to severe infection and prolonged healing. Therapies for treating skin ulcers have proven to be unsuccessful particularly in cases where the conditions causing skin ulceration remain unchanged. Accordingly, the health care industry has focused on measures to prevent the formation of skin ulcers as the best approach for treating such ulcers. In the case of decubitus ulcers, conventional prophylactic methods include, by way of example, the use of sheepskin pads, foam mattresses, specialized beds, emollients and the like. In the case of diabetic ulcers, conventional prophylactic methods include, by way of example, the use of pads in areas of nerve damage due to neuropathy (to prevent the patient from inflicting injuries to these areas due to lack of sensation), methods to enhance blood circulation in the patient, etc.

Notwithstanding such therapies, skin ulceration is a continuing and expanding problem especially with diabetic patients, bed or wheelchair ridden patients, etc, as a recent study indicates that the prevalence of pressure ulcers in hospital populations has increased by about 21% from 1989 to 1993. Meehan, *Advances in Wound Care,* Vol. 7, No. 3, pp. 27-38, 1994. Accordingly, there is an ongoing need to provide better methods for inhibiting skin ulceration and reducing the effects of pressure and irritation.

### SUMMARY OF THE INVENTION

This invention is drawn to uses of cyanoacrylate adhesives for the manufacture of cyanoacrylate adhesive formulations for inhibiting surface skin ulceration including, by way of example, decubitus ulcers (bedsores) and diabetic ulcers. The uses involve applying cyanoacrylate adhesive, particularly n-butyl cyanoacrylate adhesive, onto skin areas prone to surface skin ulceration so as to form a resilient yet flexible, waterproof polymer layer over such skin areas. In turn, this polymer layer increases the skin integrity while reducing skin irritation to the underlying skin thereby inhibiting these factors which are known to increase skin ulceration.

The uses described herein can be used by themselves to inhibit formation of surface skin ulcers, but preferably are used in conjunction with existing regimens for inhibiting formation of surface skin ulcers.

Accordingly, in one of its uses aspects, this invention is directed to the use of cyanoacrylate adhesives for the manufacture of cyanoacrylate adhesive formulations for inhibiting the formation of surface skin ulcers which method comprises:
applying to an intact skin surface area prone to ulcer formation, a sufficient amount of a cyanoacrylate adhesive so as to cover said area; and
polymerizing the cyanoacrylate adhesive so as to form a flexible, waterproof, adhesive polymer coating which adheres to the area where the adhesive was applied,
wherein the cyanoacrylate, in monomeric form, is represented by formula I: 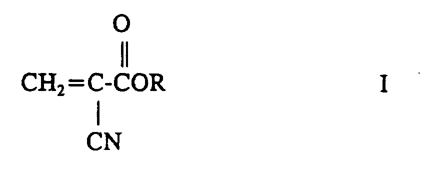 where
R is selected from the group consisting of:
   alkyl of 2 to 10 carbon atoms,
   alkenyl of 2 to 10 carbon atoms,
   cycloalkyl groups of from 5 to 8 carbon atoms,
   phenyl,
   2-ethoxyethyl,
   3-methoxybutyl,
   and a substituent of the formula: 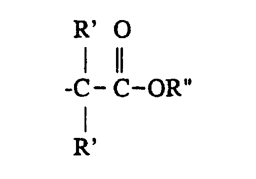 wherein each R' is independently selected from the group consisting of:
   hydrogen and methyl, and
R" is selected from the group consisting of:
   alkyl of from 1 to 6 carbon atoms,
   alkenyl of from 2 to 6 carbon atoms,
   alkynyl of from 2 to 6 carbon atoms,
   cycloalkyl of from 3 to 8 carbon atoms,
   aralkyl selected from the group consisting of benzyl, methylbenzyl and phenylethyl,
   phenyl, and
   phenyl substituted with 1 to 3 substituents selected from the group consisting of hydroxy, chloro, bromo, nitro, alkyl of 1 to 4 carbon atoms, and alkoxy of from 1 to 4 carbon atoms.

Preferably R is alkyl of from 2 to 10 carbon atoms and more preferably alkyl of from 2 to 8 carbon atoms. Even more preferably, R is butyl or octyl and most preferably, R is n-butyl.

In a preferred embodiment, the cyanoacrylate is applied at an amount of at least 0.02 milliliter (ml), and preferably from about 0.02 to about 0.2 ml, of cyanoacrylate adhesive per square centimeter of skin which is to be covered.

In another preferred embodiment, the cyanoacrylate adhesive to be applied to the skin has a viscosity of from greater than 1 to about 100 centipoise at 20°C. More preferably, the cyanoacrylate adhesive is in monomeric form and has a viscosity of from greater than 1 to about 20 centipoise at 20°C. In still another preferred embodiment, a gel having a viscosity of up to about 50,000 centipoise or more at 20°C can be used.

As used herein, the following terms have the following meanings:
The term "cyanoacrylate adhesive" refers to adhesive formulations based on cyanoacrylate monomers of formula I: 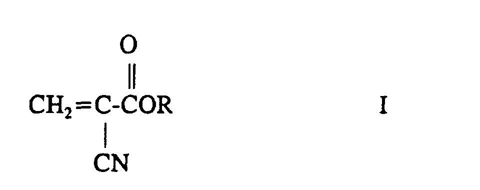
where R is selected from the group consisting of alkyl of 2 to 10 carbon atoms, alkenyl of 2 to 10 carbon atoms, cycloalkyl groups of from 5 to 8 carbon atoms, phenyl, 2-ethoxyethyl, 3-methoxybutyl, and a substituent of the formula: 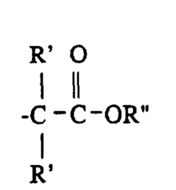 where each R' is independently selected from the group consisting of hydrogen and methyl and R" is selected from the group consisting of alkyl of from 1 to 6 carbon atoms; alkenyl of from 2 to 6 carbon atoms; alkynyl of from 2 to 6 carbon atoms; cycloalkyl of from 3 to 8 carbon atoms; aralkyl selected from the group consisting of benzyl, methylbenzyl and phenylethyl; phenyl; and phenyl substituted with 1 to 3 substituents selected from the group consisting of hydroxy, chloro, bromo, nitro, alkyl of 1 to 4 carbon atoms, and alkoxy of from 1 to 4 carbon atoms.

Preferably, R is an alkyl group of from 2 to 10 carbon atoms including ethyl, n-propyl, iso-propyl, n-butyl, iso-butyl, sec-butyl, n-pentyl, iso-pentyl, n-hexyl, iso-hexyl, 2-ethylhexyl, n-heptyl, octyl, nonyl, and decyl. More preferably, R is butyl or octyl and most preferably, R is n-butyl.

These cyanoacrylate adhesives are known in the art and are described in, for example, U.S. Patent Nos. 3,527,224; 3,591,676; 3,667,472; 3,995,641; 4,035,334; and 4,650,826.

A preferred cyanoacrylate adhesive for use in the invention is n-butyl-2-cyanoacrylate.

The cyanoacrylate adhesives described herein rapidly polymerize in the presence of moisture or tissue protein, and the n-butyl-cyanoacrylate is capable of bonding to human skin tissue without causing histoxicity or cytotoxicity.

### DETAILED DESCRIPTION OF THE PREFERRED EMBODIMENTS

This invention relates to cyanoacrylate adhesives which are useful for inhibiting formation of surface skin ulcers. The cyanoacrylate adhesive which is applied to surface skin areas prone to ulcer formation can be monomeric or partially polymeric. In general, partially polymerized cyanoacrylate adhesives are liquid polymers having a higher viscosity than that of the corresponding monomer and, therefore, are better suited for those applications which are intended to be specific for a particular skin area. In other words, less viscous materials are more likely to "run" (i.e., flow) into areas where application was not intended.

The cyanoacrylate adhesives used herein preferably have a viscosity of from greater than 1 to about 100 centipoise and more preferably from greater than 1 to about 20 centipoise at 20°C (e.g., for spray application). It is contemplated, however, that adhesives of higher viscosity such as pastes and gels having viscosities of up to 50,000 centipoise at 20°C can also be employed and will make easier skin application.

The specific viscosity of the formulation depends, in part, on the amount and degree of partially polymerized cyanoacrylate adhesive employed as well as additives which are employed to the formulation to enhance or decrease viscosity. Such factors are readily ascertainable by the skilled artisan. For example, methods for preparing partially polymerized cyanoacrylate adhesives are disclosed, for example, by Rabinowitz, U.S. Patent No. 3,527,224. Additives which can be incorporated into the formulation to enhance its viscosity include polymers such as polymethyl methacrylate (PPMA) and polymerized cyanoacrylate adhesives as disclosed in U.S. Patent Nos. 3,654,239 and 4,038,345.

Monomeric forms of cyanoacrylate adhesives are often preferred where application is to be made to a large surface area. This preference results from the fact that those forms are less viscous and, accordingly, will permit more facile large surface area application. Mixtures of monomeric forms of cyanoacrylate adhesive and partially polymerized forms of cyanoacrylate adhesive can also be used to prepare a formulation having intermediate viscosities.

For purposes of this invention, monomeric or partially polymerized n-butyl-2-cyanoacrylate is a particularly preferred adhesive and is capable of effectively bonding to human skin tissue without causing histoxicity or cytotoxicity.

Upon contact with skin moisture and tissue protein, the cyanoacrylate adhesives will polymerize or, in the case of partially polymerized cyanoacrylate adhesives, will further polymerize, at ambient conditions (skin temperature) over about 10 to 60 seconds to provide a solid layer which forms over and strongly adheres to the surface of the skin. The resulting adhesive polymer layer or coating is flexible and waterproof thereby forming a protective barrier film (layer) which increases underlying skin integrity and reduces irritation to the surface skin area arising from shearing forces, moisture, friction, etc. In turn, increases in skin integrity and reduction in irritation inhibit formation of surface skin ulcers.

Surface skin areas prone to ulcer formation are readily identifiable to the skilled medical care provider. In a preferred embodiment, such skin areas are identified as areas of nutrient deprived and irritated skin. This latter characteristic is identified by reddening of the nutrient deprived skin. Reddening of nutrient deprived skin is viewed by the medical care provider as a likely point of skin ulceration. In another preferred embodiment, surface skin areas prone to ulcer formation correspond to skin areas over bony prominences (e.g., heels, knees, elbows, shoulders, etc.) on high risk patients as identified by conventional screening tests (e.g., the Norton scale, the Braden scale, etc.)

The cyanoacrylate adhesive is applied to provide an effectively thick coating over the human skin tissue prone to surface ulcer formation.

Generally, the cyanoacrylate adhesive provides an adhesive coating over the entire skin area prone to surface skin ulceration which, when set, is waterproof and satisfactorily flexible and adherent to the tissue without peeling or cracking. Preferably, the adhesive coating has a thickness of less than about 0.5 millimeter (mm), and more preferably the coating has a thickness of less than about 0.3 mm. In a particularly preferred embodiment, the thickness of the adhesive coating is from 0.1 millimeter to 0.5 millimeter and even more preferably from 0.1 millimeter to 0.3 millimeter.

The adhesive coating can be formed by applying at least about 0.02 ml of cyanoacrylate adhesive per square centimeter of skin, more preferably from 0.02 to 0.2 ml, and still more preferably from 0.02 to 0.1 ml, of cyanoacrylate adhesive per square centimeter of skin and yet more preferably from 0.02 to 0.05 ml of cyanoacrylate adhesive per square centimeter of skin.

### FORMULATIONS

The cyanoacrylate adhesive formulations employed herein generally comprise monomeric and/or partially polymerized compounds of formula I described above and are sometimes referred to herein as simply cyanoacrylate adhesives. These formulations are liquid in nature and, upon contact with surface skin proteins and moisture, will polymerize to provide a solid film or layer over the skin surface.

The formulations may additionally contain one or more optional additives such as colorants, perfumes, plasticizers, antidiffusion agents, modifying agents and stabilizers. In practice, each of these optional additives should be both miscible and compatible with the cyanoacrylate adhesive. Compatible additives are those that do not prevent the use of the cyanoacrylate adhesives for their intended use.

In general, colorants are added so that the polymerized film will contain a discrete and discernable color. Perfumes are added to provide a pleasant smell to the formulation. Stabilizers, such as sulfur dioxide, are added to minimize *in situ* polymerization in containers during storage. Plasticizers, such as dioctylphthalate or tri(p-cresyl)phosphate, are added in order to enhance the flexibility of the resulting polymer layer. Each of these additives is conventional. For example, suitable stabilizers are disclosed in U.S. Patent No. 4,650,826 and suitable plasticizers are disclosed in U.S. Patent Nos. 2,784,127 and 4,444,933.

The amount of each of these optional additives employed in the cyanoacrylate adhesive is an amount necessary to achieve the desired effect.

The formulation is generally stored in an applicator for use in a single dose application (e.g., a disposable container) or for use in repeated applications. Single dose applicators include those having breakable or removable seals that prevent moisture, including atmospheric moisture, from contacting the formulation and causing premature *in situ* polymerization.

For repeated and intermittent usage, minimal exposure to atmospheric moisture is required. This can be achieved by devices having very narrow outlets and low initial dead space. One applicator for such repeated intermittent use is described in U.S. Patent No. 4,958,748.

Another applicator comprises a conventional spray applicator wherein the cyanoacrylate adhesive is sprayed onto the surface skin area prone to ulceration. The spray rate of the applicator can be controlled so that application of a metered quantity of adhesive per unit area of skin surface over a set period of time is controlled.

Still another applicator comprises a brush or solid paddle applicator wherein the cyanoacrylate adhesive is "painted" onto the surface skin area prone to skin ulceration.

A preferred applicator for repeated and intermittent usage is an applicator suitable for the non-sterile storage and metered dispersement of a cyanoacrylate adhesive after opening of the applicator wherein the applicator is characterized as having a resealable opening of no more than about 0.05 square inch (0.323 square centimeters) so as to permit the metered dispersement of the adhesive from the applicator and which is capable of multiple administrations of the adhesive and is further characterized as having resealing means such as a cap which either tightly mates with the applicator or which screws onto the applicator.

Preferably, the opening of the applicator is 0.001 to 0.01 square inch (0.00645 to 0.0645 square centimeters).

In another preferred embodiment, the walls of the applicator are made of a pliable material, so that upon application of pressure onto the walls, the walls depress sufficiently to force the adhesive contained in the applicator through the opening. In still another preferred embodiment, the adhesive is released from the applicator by a gravity feed methods well known in the art. Such methods do not require application of pressure to the walls of the container.

Preferably, the applicator is manufactured with its opening covered by a metal foil or other similar construction which closes this opening until the device is ready for use. The opening is then reinstated by use of a pin or similar device which punctures the covering.

Such devices for intermittent use enable multiple uses of the cyanoacrylate adhesive at different points in time by the same or different individuals.

In applicators suitable for repeated intermittent uses, the cyanoacrylate adhesive is stored at ambient conditions and is selected to be bacteriostatic. See, for example, Rabinowitz et al., U.S. Patent No. 3,527,224. When the selected adhesive is bacteriostatic, prolonged storage at ambient conditions can be achieved without regard to the sterility of the formulation because there is no adverse buildup of bacteria during storage.

### METHODOLOGY

The above-described formulations are applied to unbroken surface skin areas prone to ulceration under conditions suitable for polymerizing the adhesive so as to form a protective coating and typically under non-sterile conditions. In general, sufficient amounts of cyanoacrylate adhesive are employed to cover (i.e., coat) the entire surface skin area prone to ulceration (e.g., the entire reddened area in nutrient deprived skin). The coating is preferably extended by at least about 1 centimeter and preferably by at least about 5 centimeters beyond the surface skin area prone to ulceration (e.g., beyond the reddened area described above). However, application to broke skin is preferably avoided.

The adhesive polymer coating should be maintained in a unbroken manner over the entire skin area prone to ulceration. This can be assured by careful application of the adhesive onto the skin. Additionally, the use of a plasticizer will facilitate the maintenance of the polymer coating in an unbroken manner. However, to further ensure that the polymer coating is maintained unbroken, after the initial layer of adhesive has cured to provide for an adhesive polymer coating, a second, preferably thinner, layer is preferably applied over the adhesive polymer coating. Additional amounts of cyanoacrylate adhesive can be applied as needed to maintain an unbroken callous like covering over the ulcer prone surface skin areas.

Sufficient cyanoacrylate adhesive is preferably employed to form a coating of less than about 0.5 mm thick and more preferably at least about 0.1 mm thick. Such coatings can be formed by applying, for example, about 0.02 ml of cyanoacrylate adhesive per square centimeter of skin surface area.

The amount of cyanoacrylate adhesive applied onto the skin surface area can be controlled by the amount of adhesive packaged in a single dose product or by use of a multiple use dispenser which governs the amount of material applied onto a unit area of surface skin. In this regard, the dispenser described by Otake, U.S. Patent No. 4,958,748, is particularly advantageous because it dispenses the adhesive in a controlled drop wise manner. Other methods for the controlled dispersement of the cyanoacrylate adhesive are as described above including, by way of example, a conventional spray applicator, a brush or solid paddle applicator, and the like.

Upon application of the cyanoacrylate adhesive, the surface skin moisture, tissue protein, and temperature are sufficient to initiate polymerization of the adhesive upon application.
Thereafter, the skin surface is maintained under suitable conditions to allow polymerization to proceed to formation of an adhesive coating.

In general, the particular length of time required for polymerization will vary depending on factors such as the amount of adhesive applied, the temperature of the skin, the moisture content of the skin, the surface area of skin for adhesive application, and the like. However, in a preferred embodiment, polymerization is generally complete within 10 to 60 seconds while the skin is maintained at ambient conditions. During this period, the person to whom application of the cyanoacrylate adhesive has been made merely allows the adhesive to form a coating while minimizing any action to prevent the adhesive from being dislodged from that portion of the skin where it was applied or to adhere to unintended objects. Excess adhesive polymer can be removed with acetone (nail polish remover) which can be readily conducted except in the case where the adhesive polymer binds to a sensitive skin part (e.g., the eye lids) where it should be removed by a health care professional.

After the adhesive coating has formed, the coating strongly adheres to the skin, is flexible and waterproof, thereby forming a protective coating which enhances the integrity of the underlying skin and protects the skin from further irritation thereby retarding or inhibiting surface skin ulceration.

In general, the coating will adhere to the skin for a period of about 1-3 days after which time it sloughs off. Additional applications can be made if desired.

Because the cyanoacrylate adhesive polymer coating is waterproof, the patient is not prevented from bathing and other activities involving exposure to water during the period the adhesive layer protects this skin area.

The following examples illustrate certain embodiments of the invention.

### EXAMPLES

### Example 1

A cyanoacrylate adhesive formulation is prepared in monomeric form using n-butyl α-cyanoacrylate and which contains a colorant to readily ascertain where the formulation has been applied, 20 weight percent of dioctyl phthalate which acts as a plasticizer to enhance the flexibility of the resulting polymer composition, and 200 parts per million (ppm) of sulfur dioxide which acts as a stabilizer. The formulation is applied onto an approximately 50 square centimeter nutrient deprived and irritated skin area prone to surface skin ulceration which, in this case, is prone to decubitus ulcer formation on the buttocks of a bed ridden human male patient as evidenced by reddening of this skin indicating irritation. The formulation is applied at the rate of 0.03 milliliter per square centimeter of treated skin. Application is made by use of a latex gloved finger or by use of a finger cot. After application, the skin is maintained at ambient condition until a polymer coating forms in about 30 to 60 seconds. After the polymer coating has formed, preventive methods to inhibit decubitus ulcer formation in the patient, including, sheepskin pads, specialized beds, patient rotation and the like, are continued.

In this regard, the application of the cyanoacrylate adhesive polymer layer to the skin area prone to decubitus ulcer formation in conjunction with other conventional therapies to prevent decubitus ulcer formation will reduce the incidence of decubitus ulcer formation as compared to treatments involving conventional therapies without application of the adhesive polymer layer.

### Example 2

A cyanoacrylate adhesive formulation is prepared in monomeric form using n-butyl α-cyanoacrylate and which contains a colorant to readily ascertain where the formulation has been applied, 20 weight percent of dioctyl phthalate which acts as a plasticizer to enhance the flexibility of the resulting polymer composition, and 200 parts per million (ppm) of sulfur dioxide which acts as a stabilizer. The formulation is applied onto an approximately 20 square centimeters nutrient deprived and irritated skin area prone to surface skin ulceration which, in this case, is prone to diabetic ulcer formation on the foot of a human female patient as evidenced by reddening of this skin indicating irritation. The formulation is applied at an amount of 0.03 milliliter per square centimeter of treated skin and, after application, the skin is maintained at ambient condition until a polymer coating forms in about 30 to 60 seconds. After the polymer coating has formed, preventive methods to inhibit diabetic ulceration in this patient, including, the use of pads, specialized footwear, methods to enhance blood circulation in the patient, and the like, are continued.

In this regard, the application of the cyanoacrylate adhesive polymer layer to the skin area prone to diabetic ulceration in conjunction with other conventional therapies to inhibit diabetic ulceration will reduce the incidence of diabetic ulceration as compared to treatments involving conventional therapies without application of the adhesive polymer layer.

### Example 3

An evaluation of the effect of n-butyl cyanoacrylate adhesive on the inhibition of decubitus ulcers in 17 adult patients located in nine different nursing homes was conducted. In this example, 17 patients were selected based on a history of recurring decubitus ulcer formation or, based on medical evaluation, were deemed to be at high risk of developing decubitus ulcers. A prophylactic treatment regimen was then employed on these patients which regimen consisted of using conventional pressure ulcer preventative practices primarily involving periodic rotation of the patient as well as application of an n-butyl cyanoacrylate adhesive composition onto the intact skin areas which appeared likely to develop pressure ulcers by virtue of irritation and reddening of the skin or unbroken blister formation which is classified as a stage I decubitus ulcer or by application of the n-butyl cyanoacrylate composition onto intact skin adjacent to type II decubitus ulcers. Application of n-butyl cyanoacrylate consisted of application of about 1-5 drops of the n-butyl cyanoacrylate composition onto the surface skin area prone to ulceration wherein each drop corresponds to about 0.03 ml of cyanoacrylate.

The specific amount of n-butyl cyanoacrylate adhesive and point of application of the adhesive for each patient are set forth in the following Table I:

**TABLE I**

| APPLICATION OF N-BUTYL CYANOACRYLATE ADHESIVES TO PATIENTS TO INHIBIT DECUBITUS ULCER FORMATION | | | | | |
|---|---|---|---|---|---|
| NURSING HOME | PATIENT NUMBER | WHERE | TYPE OF IRRITATION | SIZE | AVE. NUMBER OF DROPS |
| A | 1 | Heel | Redness | 3 cm | 2 |
| A | 2 | Heel | Redness | 3 cm | 2 |
| B | 3 | Heels(2) | Blister (Stage I) | 3-4 cm | 2 |
| B | 4 | Heels(2) | Redness | 3-5 cm | 2 |
| | | Elbows(2) | Redness | 3-5 cm | 2 |
| C | 5 | Cheek | Redness | ½-3 in | 2 |
| C | 6 | Lower Leg | Bruise | 2-3 in | 4-5 |
| D | 7 | Shoulder | Bony Prominence | 3-4 cm | 3-4 |
| D | 8 | Hip | Bony Prominence | 3-4 cm | 3-4 |
| E | 9 | Heels(2) | Redness | 5 cm | 1 |
| E | 10 | Hip | Blister (Stage I) | 3-4 cm | 2 |
| E | 11 | Top of Foot | Redness | 2-5 cm | 1 |
| F | 12 | Heel | Redness (Scabbed over --healed stage II) | 6-8 cm | 3 |
| G | 13 | Knees & Heels | Redness | 2.5 cm | 3 |
| G | 14 | Heel | Skin Redness around Stage II ulcer | 2.5 cm | 3 |
| G | 15 | Heels(2) | Redness | 2.5 cm | 3 |
| H | 16 | Hip | Redness (healed Stage II ulcer) | 2 cm | 2-3 |
| I | 17 | Gluteal Fold | Stage I area around stage II | 3-3.5 cm | 3-4 |

After application, the cyanoacrylate adhesive was allowed to form a polymeric film and, on average, reapplication of the adhesive was conducted every 24 hours until the evaluation was completed.

As a result of this evaluation, all patients treated with conventional pressure ulcer preventative practices as well as n-butyl cyanoacrylate adhesive having either redness/irritation of intact skin or having blistering of intact skin characterized as stage I decubitus ulcer formation did not progress to stage II decubitus ulcer formation characterized by broken skin. Moreover, all patients treated with conventional pressure ulcer preventative practices as well as n-butyl cyanoacrylate adhesive in skin areas adjacent to stage II decubitus ulcer formation showed minimal expansion of the ulcer to the adjacent skin areas. The specific results documented for each of the seventeen patients regarding the use of the n-butyl cyanoacrylate adhesive are set forth in Table II below:

**TABLE II**

| RESULTS OF APPLICATION OF N-BUTYL CYANOACRYLATE ADHESIVES TO PATIENTS TO INHIBIT DECUBITUS ULCER FORMATION | |
|---|---|
| PATIENT NO. | RESULT |
| 1 | NO BREAKDOWN OF SKIN ON HEEL |
| 2 | LESS PERSPIRATION AND NO IRRITATION |
| 3 | NO BREAKDOWN OF BLISTER TO STAGE II DECUBITUS ULCER |
| 4 | NO SKIN BREAKDOWN ON HEELS OR ELBOWS |
| 5 | NO IRRITATION DEVELOPED |
| 6 | ABSORPTION OF BRUISE WITH NO SKIN BREAKDOWN |
| 7 | NO IMPROVEMENT/NO CHARGE BUT SKIN DID NOT BREAKDOWN |
| 8 | NO SKIN BREAKDOWN |
| 9 | NO IRRITATION/REDDENING LEFT |
| 10 | WARMING SENSATION UPON APPLICATION--TREATMENT STOPPED |
| 11 | NO IRRITATION/REDDENING LEFT |
| 12 | REDNESS HEALED UP |
| 13 | IRRITATION IMPROVED BUT PATIENT PASSED AWAY DUE TO OTHER CONDITIONS |
| 14 | NO IRRITATION EVIDENT--HEELS ALMOST HEALED |
| 15 | PATIENT'S HEELS LOOK BETTER, ALMOST TOTALLY HEALED |
| 16 | NO FURTHER BREAKDOWN, REDDENING STILL THERE BUT PROGRESSION SEEMS TO HAVE STOPPED |
| 17 | AREA IS RESOLVED, NO OPEN STAGE II'S AND STAGE I ALMOST GONE (NO REDNESS) |

The above results illustrating the failure of any patient to progress to stage II decubitus ulcer evidences the fact that the application of cyanoacrylate adhesive in conjunction with conventional pressure ulcer preventative practices inhibits decubitus ulcer formation.

From the foregoing description, various modifications and changes in the composition and method will occur to those skilled in the art.

## Claims

1. Use of a cyanoacrylate composition which comprises monomeric and/or partially polymerized cyanoacrylate which in monomeric form is represented by formula (I) 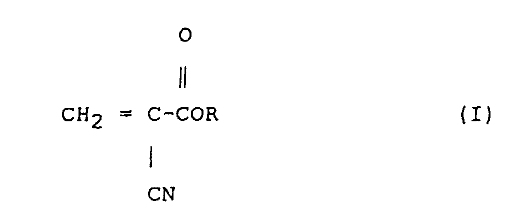 where R is selected from the group consisting of :
- alkyl of 2 to 10 carbon atoms,
- alkenyl of 2 to 10 carbon atoms,
- cycloalkyl groups of from 5 to 8 carbon atoms,
- phenyl,
- 2-ethoxyethyl
- 3-methoxybutyl,
- and a subsituent of the formula : 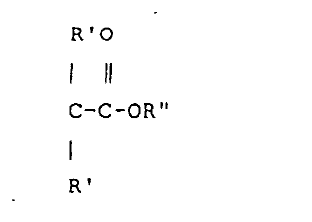
wherein each
R' is independently selected from the group consisting of :
- hydrogen and methyl, and
R" is selected from the group consisting of :
- alkyl of from 1 to 6 carbon atoms,
- alkenyl of from 2 to 6 carbon atoms,
- alkynyl of from 2 to 6 carbon atoms,
- cycloalkyl of from 3 to 8 carbon atoms,
- aralkyl selected from the group consisting of benzyl, methylbenzyl and phenylethyl,
- phenyl, and phenyl, and
- phenyl substitued with 1 to 3 substituents selected from the group consisting of hydroxy, chloro, bromo, nitro, alkyl of 1 to 4 carbon atoms, and alkoxy of from 1 to 4 carbon atoms, for the manufacture of a cyanocrylate adhesive formulation for inhibiting the formation of surface skin ulcers selected from the group consisting of decubitus ulcers and diabetic ulcers, in a patient employing conventional prophylactic treatment to inhibit surface skin ulcer formation in conjunction, by applying to an unbroken skin surface area prone to ulceration, a sufficient amount of the cyanocrylate adhesive formulation so as to cover said area and polymerizing the cyanoacrylate adhesive formulation so as to form a flexible, waterproof, adhesive polymer coating which adheres to the area where the adhesive formulation was applied.

2. The use according to claim 1, wherein R is alkyl of from 2 to 10 carbon atoms.

3. The use according to claim 2, wherein R is butyl or octyl.

4. The use according to claim 3, wherein R is n-butyl.

5. The use according to claim 1, wherein the formulation is able to provide at least 0.02 ml of cyanoacrylate adhesive per square centimeter of skin which is to be covered.

6. The use according to claim 5, wherein the formulation is able to provide from 0.02 ml to 0.5 ml per square centimeter of skin which is to be covered.

7. The use according to claim 6, wherein the formulation is able to be applied at an amount of from 0.02 ml to 0.05 ml per square centimeter of skin which is to be covered.

8. The use according to claim 1, wherein the formulation has a viscosity of from greater than 1 to about 100 centipoise at 20°C.

9. The use according to claim 8, wherein the formulation has a viscosity of from greater than 1 to 20 centipoise at 20°C.

10. The use according to claim 1, wherein the formulation is contained in a single use disposable applicator.

11. The use according to claim 1, wherein the formulation is contained in a multiple, intermittent use applicator.

12. The use according to claim 1, wherein said ulcers are decubitus ulcers.

13. The use according to claim 1, wherein the surface skin ulcers are diabetic ulcers.

## Patentansprüche

1. Verwendung einer Cyanoacrylatzusammensetzung, die monomeres und/oder teilweise polymerisiertes Cyanoacrylat umfasst, welches in monomerer Form dargestellt wird durch die Formel (I) 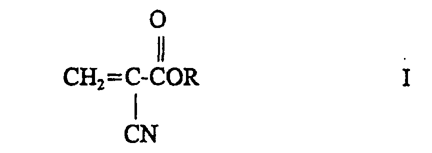 worin R ausgewählt wird aus der Gruppe bestehend aus:
- Alkyl aus 2 bis 10 Kohlenstoffatomen,
- Alkenyl aus 2 bis 10 Kohlenstoffatomen,
- Cycloalkyl-Gruppen aus von 5 bis zu 8 Kohlenstoffatomen,
- Phenyl,
- 2-Ethoxyethyl
- 3-Methoxybutyl
- und einem Substituenten mit der Formel: 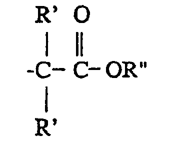
worin
R' jeweils unabhängig voneinander ausgewählt wird aus der Gruppe bestehend aus:
- Wasserstoff und Methyl, und
R" ausgewählt wird aus der Gruppe bestehend aus :
- Alkyl aus von 1 bis zu 6 Kohlenstoffatomen,
- Alkenyl aus von 2 bis zu 6 Kohlenstoffatomen,
- Alkynyl aus von 2 bis zu 6 Kohlenstoffatomen,
- Cycloalkyl aus von 3 bis zu 8 Kohlenstoffatomen,
- Aralkyl ausgewählt aus der Gruppe bestehend aus Benzyl, Methylbenzyl und Phenylethyl,
- Phenyl, und
- Phenyl substituiert mit 1 bis 3 Substituenten ausgewählt aus der Gruppe bestehend aus Hydroxy, Chlor, Brom, Nitro, Alkyl aus 1 bis 4 Kohlenstoffatomen, und Alkoxy aus 1 bis 4 Kohlenstoffatomen, zur Herstellung einer Cyanoacrylat-Klebstoff-Formulierung zur Verhinderung der Bildung von oberflächlichen Hautulzera ausgewählt aus der Gruppe bestehend aus Dekubitus-Ulzera und diabetischen Ulzera, bei einer konventionellen prophylaktischen Behandlung in der Patientenbetreuung zur Verhinderung der Ulkusbildung der Hautoberfläche in Verbindung mit, durch Auftragen auf einen unversehrten Bereich der Hautoberfläche mit der Neigung zur Ulzeration, einer Menge der Cyanoacrylat-Klebstoff-Formulierung ausreichend, um diesen Bereich abzudecken, und Polymerisation der Cyanoacrylat-Klebstoff-Formulierung, um eine flexible, wasserdichte, adhäsive, polymere Abdeckung zu bilden, die an dem Bereich anhaftet, wo die Klebstoffverbindung angewendet wurde.

2. Die Verwendung nach Anspruch 1, worin R ein Alkyl aus 2 bis zu 10 Kohlenstoffatomen ist.

3. Verwendung nach Anspruch 2, worin R Butyl oder Octyl ist.

4. Verwendung nach Anspruch 3, worin R n-Butyl ist.

5. Verwendung nach Anspruch 1, worin die Formulierung mindestens 0,02 ml Cyanoacrylatklebstoff pro Quadratzentimeter Haut, die zu bedecken ist, liefern kann.

6. Verwendung nach Anspruch 5, worin die Formulierung in der Lage ist, von 0,02 ml bis 0,5 ml pro Quadratzentimeter Haut, die zu bedecken ist, zu liefern.

7. Verwendung nach Anspruch 6, worin die Formulierung angewendet werden kann in einer Menge von 0,02 ml bis 0,05 ml pro Quadratzentimeter Haut, die bedeckt werden muss.

8. Verwendung nach Anspruch 1, worin die Formulierung eine Viskosität von höher als 1 bis etwa 100 Zentipoise bei 20°C hat.

9. Verwendung nach Anspruch 8, worin die Formulierung eine Viskosität von höher als 1 bis zu 20 Zentipoise bei 20°C hat.

10. Verwendung nach Anspruch 1, worin die Formulierung in einem Wegwerfapplikator zum einmaligen Gebrauch enthalten ist.

11. Verwendung nach Anspruch 1, worin die Formulierung in einem Applikator zum mehrfachen, unterbrochenen Gebrauch enthalten ist.

12. Verwendung nach Anspruch 1, worin die Ulzera Dekubitus-Ulzera sind.

13. Verwendung nach Anspruch 1, worin die Ulzera der Hautoberfläche diabetische Ulzera sind.

## Revendications

1. Utilisation d'une composition de cyanoacrylate qui comprend un cyanoacrylate monomère et/ou partiellement polymérisé qui, sous forme monomère, est représenté par la formule (I) 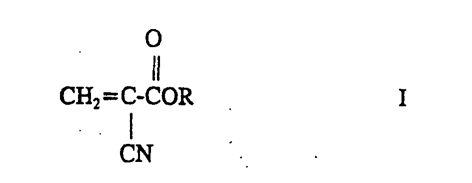 où R est choisi dans le groupe constitué par:
- alkyle de 2 à 10 atomes de carbone,
- alcényle de 2 à 10 atomes de carbone,
- les groupes cycloalkyles de 5 à 8 atomes de carbone,
- phényle,
- 2-éthoxyéthyle,
- 3-méthoxybutyle,
- et un substituant de formule: 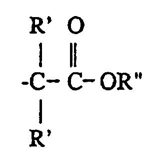
dans laquelle chaque
R' est indépendamment choisi dans le groupe constitué par:
- hydrogène et méthyle, et
R" est choisi dans le groupe constitué par:
- alkyle de 1 à 6 atomes de carbone,
- alcényle de 2 à 6 atomes de carbone,
- alcynyle de 2 à 6 atomes de carbone,
- cycloalkyle de 3 à 8 atomes de carbone,
- aralkyle choisi dans le groupe constitué par benzyle, méthylbenzyle et phényléthyle,
- phényle, et
- phényle substitué par 1 à 3 substituants choisis dans le groupe constitué par hydroxy, chloro, bromo, nitro, alkyle de 1 à 4 atomes de carbone, et alcoxy de 1 à 4 atomes de carbone,
pour la fabrication d'une formulation adhésive cyanoacrylate pour inhiber la formation d'ulcères cutanés de surface choisis dans le groupe constitué par les escarres de décubitus et les ulcères diabétiques, chez un patient suivant un traitement prophylactique traditionnel pour inhiber la formation d'ulcères cutanés de surface conjointement, en appliquant sur une aire cutanée de surface sans discontinuité ayant une tendance à l'ulcération une quantité suffisante de la formulation adhésive cyanoacrylate de façon à recouvrir ladite aire et en polymérisant la formulation adhésive cyanoacrylate de façon à former un revêtement polymère adhésif flexible imperméable à l'eau qui adhère à l'aire où la formulation adhésive a été appliquée.

2. Utilisation selon la revendication 1, dans laquelle R est alkyle de 2 à 10 atomes de carbone.

3. Utilisation selon la revendication 2, dans laquelle R est butyle ou octyle.

4. Utilisation selon la revendication 3, dans laquelle R est n-butyle.

5. Utilisation selon la revendication 1, dans laquelle 1a formulation est apte à fournir au moins 0,02 ml d'adhésif cyanoacrylate par centimètre carré de peau qui doit être recouverte.

6. Utilisation selon la revendication 5, dans laquelle la formulation est apte à fournir de 0,02 ml à 0,5 ml par centimètre carré de peau qui doit être recouverte.

7. Utilisation selon la revendication 6, dans laquelle la formulation est apte à être appliquée en une quantité de 0,02 ml à 0,05 ml par centimètre carré de peau qui doit être recouverte.

8. Utilisation selon la revendication 1, dans laquelle la formulation a une viscosité de plus de 1 jusqu'à environ 100 cP à 20°C.

9. Utilisation selon la revendication 8, dans laquelle la formulation a une viscosité de plus de 1 jusqu'à 20 cP à 20°C.

10. Utilisation selon la revendication 1, dans laquelle la formulation est contenue dans un applicateur jetable à usage unique.

11. Utilisation selon la revendication 1, dans laquelle la formulation est contenue dans un applicateur à usage multiple intermittent.

12. Utilisation selon la revendication 1, dans laquelle lesdits ulcères sont des escarres de décubitus.

13. Utilisation selon la revendication 1, dans laquelle les ulcères cutanés de surface sont des ulcères diabétiques.
